# EUROPÄISCHE PATENTANMELDUNG

(11) **EP 1 308 477 A1**
(43) Veröffentlichungstag der Anmeldung: **07.05.2003**
(21) Anmeldenummer: 02022557.9
(22) Anmeldetag: 08.10.2002
(51) Int. Cl.: C08K 5/00, C08L 89/06, C09K 11/06, C08J 3/24, A61K 6/08, C08F 2/50

(54) **Lichthärtendes Kunststoffmaterial mit Indikator zur Anzeige des Aushärtungsgrades**

(30) Priorität: 06.11.2001 DE 10154420
(71) Anmelder: Carl von Ossietzky Universität Oldenburg, 26129 Oldenburg (DE)
(72) Erfinder: Reuter, Rainer, Dr., 26131 Oldenburg (DE); Weisel, Christine, 21107 Hamburg (DE)
(74) Vertreter: Eisenführ, Speiser & Partner

(57) **Zusammenfassung**

Beschrieben wird ein lichthärtendes Kunststoffmaterial, umfassend ein Gemisch aus:
- einem lichthärtenden Kunstharz und
- einem Farbstoff, dessen Absorptions- und/oder Fluoreszenzverhalten vom Aushärtungsgrad des Kunstharzes abhängen.

## Beschreibung

Die vorliegende Erfindung betrifft primär (a) ein lichthärtendes Kunststoffmaterial, das ein lichthärtendes Kunstharz umfaßt. Insbesondere betrifft die Erfindung solche lichthärtenden Kunststoffmaterialien, die bei der Herstellung von Linern (Inliner, Reliner), d.h. bei der Sanierung von schadhaften (Alt-)Rohren eingesetzt werden können.

Die Erfindung betrifft zudem (b) einen lichthärtenden Liner zur Auskleidung von Rohren, (c) ein entsprechendes System zur Auskleidung von Rohren, (d) ein Verfahren zur Bestimmung des Aushärtungsgrades eines lichthärtenden Kunstharzes (beispielsweise bei dessen Verwendung in einem Liner) sowie (e) ein Verfahren zur Aushärtung eines lichthärtenden Liners.

Zur Sanierung von schadhaften, insbesondere nicht begehbaren Rohrleitungen werden in zunehmendem Maße Relining-Verfahren eingesetzt. Hierbei wird ein mit härtbarem (beispielsweise lichthärtendem) Kunststoffmaterial imprägnierter Schlauch in ein zu sanierendes Rohr eingezogen, zum Beispiel mit Druckluft aufgestellt (d.h. an die Altrohrwandung angepreßt) und dann ausgehärtet, zum Beispiel unter Einsatz einer innerhalb des aufgestellten Inliners (Schlauches) verfahrbaren Beleuchtungseinheit, welche zur Aushärtung des lichthärtenden Kunststoffmaterials geeignete Strahlung (häufig im UV-Bereich) auf die Inliner-Innenwandung appliziert. Unter dem Einfluß des eingestrahlten Lichts härtet das lichthärtende Kunststorfmaterial dann in gewünschter Weise aus.

In der WO 99/05520 (und der DE 197 39 145 C1) ist bereits auf Schwierigkeiten hingewiesen worden, die auftreten können, wenn eine Aushärteeinrichtung (zum Beispiel also eine Beleuchtungseinheit) mit gleichbleibender Geschwindigkeit durch den Inliner (Schlauch) gezogen werden soll, um auf der gesamten Länge des Inliners eine gleichmäßige Aushärtung zu bewirken. Treten nämlich im zu sanierenden Altrohr plötzliche Durchmesservariationen auf, kann dies zu kurzzeitigen Änderungen der Fahrgeschwindigkeit der Aushärteeinrichtung führen oder aber beispielsweise zu einer ungleichmäßigen Belichtung der Inliner-Innenwandung. In jedem Fall ist eine gleichmäßige Aushärtung des Inliners nicht mehr garantiert und dieser kann nach Abschluß der Sanierungsarbeiten Undichtigkeiten aufweisen. Aus den genannten Patentdokumenten ist ein Verfahren zur Messung des Aushärtungsgrades eines Inliners bekannt, bei dem elektromagnetische Strahlung (vorzugsweise UV-Licht) auf die Inliner-Innenwandung gerichtet und ihre Reflexion und/oder Transmission durch die Wandung gemessen wird. Hierbei wird von der physikalischen Gegebenheit Gebrauch gemacht, dass sich die Transmission aushärtbarer Materialien bezüglich elektromagnetischer Strahlung (wie zum Beispiel UV-Licht) mit dem Aushärtegrad des Materials ändert und deshalb durch Messen des mit dem Inliner (Schlauch) in Wechselwirkung getretenen reflektierten Strahlungsanteils auf den Aushärtegrad des Inliners rückgeschlossen werden kann.

Als aushärtbare Materialien sollen gemäß der WO 99/05520 (DE 197 39 145 C1) solche gewählt werden, bei denen sich zum Beispiel die Absorption der aushärtbaren Schlauchwand im Wellenlängenbereich der verwendeten elektromagnetischen Strahlung während des Aushärtens deutlich ändert; es werden jedoch keine Materialbeispiele genannt. Stattdessen erfolgt der Hinweis, dass dem aushärtbaren Material lichtempfindliche Bestandteile (zum Beispiel Pigmente) beigefügt werden können, die zwar zum Aushärtevorgang selbst nicht beitragen, jedoch dadurch, dass sie der elektromagnetischen Strahlung ausgesetzt werden, sich verfärben und so den Transmissionsgrad verändern.

In eigenen Untersuchungen hat sich nun gezeigt, dass sich das Absorptionsverhalten der üblicherweise in Inlinern eingesetzten lichthärtenden Kunststoffmaterialien im Wellenlängenbereich der zur Aushärtung verwendeten elektromagnetischen Strahlung während des Aushärtens nur unzureichend ändert. Das Aushärten führt somit nur zu einem unpräzise messbaren Effekt, der den in Industrieländern hohen Anforderungen an die Qualitätssicherung nicht genügt. Der Einsatz lichtempfindlicher Pigmente (wie in der WO 99/05520 vorgeschlagen) würde andererseits aufgrund von deren hohen Eigenkosten zu einer Verteuerung des Inliner-Verfahrens führen, die nicht in allen Fällen akzeptabel erscheint.

Es war daher die (primäre) Aufgabe der vorliegenden Erfindung, ein lichthärtendes Kunststoffmaterial anzugeben, dessen optische Eigenschaften sehr deutlich vom Aushärtungsgrad des Kunststoffmaterials abhängen. Dieses lichthärtende Kunststoffmaterial sollte vorzugszweise zum Einsatz in Linern zur Sanierung von schadhaften Altrohren geeignet sein.

Erfindungsgemäß wird diese (primäre) Aufgabe durch Angabe eines lichthärtenden Kunststoffmaterials gelöst, welches ein Gemisch aus
- einem lichthärtenden Kunstharz und
- einem Farbstoff umfasst, dessen Absorptions- und/oder Fluroreszenzverhalten vom Aushärtungsgrad des Kunstharzes abhängen.

Die Erfindung beruht auf der überraschenden Erkenntnis, dass bestimmte Farbstoffe, die ihr Absorptions- und/oder Fluoreszenzverhalten durch Bestrahlung mit Licht einer Wellenlänge, wie sie zum Aushärten eines lichthärtenden Kunstharzes eingesetzt wird, nicht verändern, als Indikator für den Aushärtungsgrad des lichthärtenden Kunstharzes geeignet sind, wenn sie in diesen eingebettet sind. Bereits äußerst geringe Mengen dieser Farbstoffe genügen, um ein lichthärtendes Kunstharz, dessen optische Eigenschaften nur unwesentlich vom Aushärtungsgrad abhängen, in ein lichthärtendes Kunststoffmaterial zu überführen, dessen optische Eigenschaften Absorption und Fluoreszenz (innerhalb bestimmter Wellenlängenbereiche, die durch den Farbstoff bestimmt sind) überaus deutlich vom Aushärtungsgrad des Kunstharzes abhängen. Dessen Verwendbarkeit z.B. als Linermaterial wird dabei durch einen Anteil des besagten Farbstoffes nicht beeinträchtigt. Der Fachmann wird Sorge tragen, dass der Anteil an Farbstoff in dem Gemisch mit dem lichthärtenden Kunstharz in einem Bereich liegt, der eine Bestimmung des Aushärtungsgrades des Kunstharzes durch Auswertung des Absorptions- und/oder Fluoreszenzverhaltens des Farbstoffes ermöglicht, in dem aber gleichzeitig die sonstigen mechanischen oder chemischen Eigenschaften des lichthärtenden Kunststoffmaterials denen des lichthärtenden Kunstharzes (ohne Farbstoffzusatz) im Wesentlichen unverändert entsprechen.

Vorzugsweise wird ein Farbstoff eingesetzt, welcher im sichtbaren Bereich des elektromagnetischen Wellenspektrums absorbiert, wenn das lichthärtende Kunstharz im nicht ausgehärteten Zustand vorliegt. So zeigt beispielsweise der in einer Vielzahl von Fällen geeignete Farbstoff Rhodamin B im Gemisch mit einem in Styrol gelösten ungesättigten Polyesterharz (UP-Harz) üblicherweise eine rote Farbe, die solange Bestand hat, wie das Kunstharz (UP-Harz) nicht ausgehärtet ist.

Vorteilhafterweise werden lichthärtendes Kunstharz und Farbstoff so ausgewählt, dass der Farbstoff in einem Bereich des elektromagnetischen Wellenspektrums absorbiert, der zumindest im Wesentlichen für die Lichthärtung des Kunstharzes nicht relevant ist. Wird beispielsweise für die Härtung eines lichthärtenden Kunstharzes UV-Licht benötigt, sollte der eingesetzte Farbstoff keine oder eine nur sehr geringe Absorption im UV-Bereich aufweisen.

Besonders gute Resultate wurden in eigenen Untersuchungen mit Farbstoffen erzielt, die aus der Gruppe ausgewählt sind, die aus Rhodamin B, Rhodamin 19, Kresylviolett, Nilblau und Fluorescein besteht. Diese Farbstoffe lassen sich mit den üblichen zur Herstellung von Linern eingesetzten lichthärtenden Kunstharzen verwenden, und es werden nur so geringe Mengen an diesen Farbstoffen benötigt, dass keine nennenswerte Modifikation der Eigenschaften des ausgehärteten Kunstharzes im Vergleich mit dem farbstofffreien Kunstharz zu beobachten ist.

Als lichthärtendes Kunstharz in Linern werden üblicherweise Duroplaste eingesetzt, wobei diese vorzugsweise aus der Gruppe ausgewählt sind, die aus ungesättigtem Polyesterharz (UP-Harz) und Expoxidharz (EP-Harz) besteht. In bestimmten Fällen (bei aggressiven Abwässern u.dgl.) werden vorzugsweise bestimmte Vinylesterharze eingesetzt.

Das Kunststoffmaterial kann neben dem lichthärtenden Kunstharz, ggf. einem Fotokatalysator sowie dem Farbstoff noch weitere übliche Zusatzstoffe umfassen. Dieser Fotokatalysator ist vorzugsweise so ausgewählt, dass er zur Katalyse der Aushärtungsreaktion des Kunstharzes in einem spektralen Bereich absorbiert, in dem der Indikator-Farbstoff zumindest im Wesentlichen nicht absorbiert. Auf diese Weise wird verhindert, dass der Aushärtevorgang dadurch verlängert wird, dass der Farbstoff in einem Spektralbereich absorbiert, in dem auch die Fotokatalysatoren für den Aushärtevorgang absorbieren. Es wird gewissermaßen für die Aushärtung ein Spektralbereich reserviert, und außerhalb dieses Bereiches (beispielsweise des für die UV-Härtung relevanten blauen Bereiches um 420 nm Wellenlänge) kann ein schmal- oder breitbandiges Absorptionsvermögen des Farbstoffes vorliegen. Bei Bestrahlung mit einer entsprechenden Beleuchtungseinrichtung lässt sich das rückgestreute Licht auswerten; wird durch Beleuchtung in dem für die Aushärtung reservierten Bereich das Kunststoffmaterial ausgehärtet, ändert sich das Absorptionsverhalten des Farbstoffes und verschwindet in Einzelfällen vollständig.

Ein Farbstoff, der für Fluoreszenzmessungen eingesetzt werden soll, wird vorzugsweise schmalbandig in einem Bereich des Spektrums absorbieren, in dem die Beleuchtungseinrichtung emittiert (aber vorzugsweise nicht innerhalb des für die Aushärtung reservierten Spektralbereiches); auf diese Weise wird durch Absorption eine Fluoreszenzanregung bewirkt. Die Fluoreszenzemission des Farbstoffes wird vorzugsweise in einem Bereich stattfinden, in dem die Beleuchtungseinrichtung keine oder eine nur geringe Emission zeigt, so dass das Fluroreszenzlicht vorteilhafterweise ohne einen durch die Beleuchtungseinrichtung verursachten Strahlungsuntergrund empfindlich nachgewiesen werden kann.

Bevorzugte lichthärtende Kunststoffmaterialien besitzen zusammenfassend mehrere oder sämtliche der nachfolgend aufgeführten Eigenschaften:
- Der in ihnen enthaltene Farbstoff absorbiert im Wesentlichen keine Strahlung in dem für die Härtung des Harzes besonders effizienten Spektralbereich.
- Die Farbänderung des Farbstoffes bei Aushärtung des Kunstharzes wird durch den Aushärtvorgang selbst und nicht etwa direkt durch eine Wechselwirkung mit der für die Aushärtung verantwortlichen Strahlung bewirkt.
- Das nicht mit Farbstoff versehene Harz besitzt eine nur geringe Eigenfarbe und ist im nicht ausgehärteten Zustand zumindest nahezu transparent. Bei Bestrahlung einer dickeren Schicht des lichthärtenden Kunststoffmaterials (Kunstharz + Farbstoff) lässt sich daher von der Bestrahlungsseite aus optisch kontrollieren, ob auf der nicht bestrahlten Seite der Schicht eine vollständige Aushärtung stattgefunden hat.
- Die Farbänderung des Farbstoffes im Zuge der Aushärtung des Kunstharzes ist so intensiv, dass eine optische Kontrolle des Aushärtungsgrades (a) abbildend unter Verwendung von Farb-Videokameras und/oder (b) nicht-abbildend mit Filter/Fotodetektor-Kombinationen durchgeführt werden kann. Für die Messung des Farbkontrastes sind dabei die Emissionslinien der für die Lichthärtung genutzten Beleuchtungseinrichtungen im sichtbaren Spektralbereich oftmals ausreichend, so dass zusätzliche Lampen für die Farbmessung nicht benötigt werden.
- Der Farbstoff zeigt zusätzlich zur Absorption auch eine intensive Fluoreszenzemission, welche durch den Aushärtungsvorgang modifiziert wird. Der Aushärtungsgrad des Kunststoffmaterials kann demzufolge mittels Fluoreszenzspektroskopie bestimmt werden.

Ein erfindungsgemäßer lichthärtender Liner zur Auskleidung von Rohren umfasst
- ein Trägermaterial und
- ein lichthärtendes Kunststoffmaterial der vorstehend beschriebenen Art, umfassend ein Gemisch aus:
- einem lichthärtenden Kunstharz und
- einem Farbstoff, dessen Absorptions- und/oder Fluoreszenzverhalten vom Aushärtungsgrad des Kunstharzes abhängen.

Das Trägermaterial ist dabei mit dem lichthärtenden Kunststoffmaterial imprägniert, so dass der Liner bei Aushärtung des Kunstharzes ebenfalls aushärtet. Von üblichen lichthärtenden Linern unterscheidet sich der erfindungsgemäße Liner also durch die Gegenwart des Farbstoffes.

Als Trägermaterial können insbesondere Glasfaserkomplexe (Glasfasermatten) verwendet werden, die mit dem Harz getränkt werden. Für das Glasfasergewebe werden beispielsweise Aluminiumborosilikatgläser (sogenannte E-Gläser für die Elektroindustrie) aber auch Aluminiumsilikatgläser (z.B. ECR-Gläser, d.h. Electric Corrosion Resistance-Gläser) eingesetzt. Die harzimprägnierten Glaserfasergewebe können durch innere und äußere Spezialfolien geschützt werden.

Ein erfindungsgemäßes System, das insbesondere zur Auskleidung von Altrohren geeignet ist, umfasst einen erfindungsgemäßen lichthärtenden kunstharzimprägnierten Liner, eine oder mehrere Beleuchtungseinheiten zur Emission (a) von Strahlung eines ersten Wellenlängenbereiches zur Aufhärtung des Liners und (b) von Strahlung eines zweiten Wellenlängenbereiches zur Absorption durch den Farbstoff des lichthärtenden Kunststoffmaterials und/oder zu dessen Fluoreszenz-Anregung, sowie einen Empfänger für Informationen zum Absorptions- oder Fluoreszenzverhalten des lichthärtenden Kunststoffmaterials bei Bestrahlung mit Licht des zweiten Wellenlängenbereiches. Der erste oder zweite Wellenlängenbereich sind dabei vorzugsweise nicht identisch; eine gewisse Überlappung ist jedoch gestattet, wenngleich vorzugsweise der erste und der zweite Wellenlängenbereich vollständig voneinander getrennt sind, damit die Absorption durch den Farbstoff die Aushärtung des Liners nicht beeinträchtigt.

Vorteilhafterweise umfasst das erfindungsgemäße System zur Auskleidung von Rohren zusätzlich Steuerungsmittel, in denen die Informationen zum Absorptions- oder Fluoreszenzverhalten des lichthärtenden Kunststoffmaterials zur Steuerung der Aushärtung des Liners weiter verarbeitet werden. Vorteilhafterweise sind die Steuerungsmittel so eingerichtet, dass sie die Geschwindigkeit der Fortbewegung des oder der Beleuchtungseinheiten innerhalb eines Liners steuern, z B. durch Übermitteln entsprechender Befehle an einen Wagen zum Transport des oder der Beleuchtungseinheiten innerhalb eines Liners.

In einem erfindungsgemäßen Verfahren zur Bestimmung des Aushärtungsgrades eines lichthärtenden Kunstharzes wird das Kunstharz in seinem nicht ausgehärteten Zustand (beispielsweise gelöst in einem geeigneten Lösungsmittel) mit einem Farbstoff vermischt, dessen Absorptionsund/oder Fluoreszenzverhalten vom Aushärtungsgrad des Kunstharzes abhängen und das Absorptions- und/oder Fluoreszenzverhalten des Farbstoffs wird dann bestimmt. Zur Bestimmung der zeitlichen Veränderung des Aushärtungsgrades des nicht härtenden Kunstharzes wird dementsprechend das Absorptions- und/oder Fluoreszenzverhalten des Farbstoffes über einen längeren (Aushärtungs-)-Zeitraum optisch erfasst. Die Auswertung der erhaltenen Informationen kann zur Steuerung der Vorschubgeschwindigkeit einer Beleuchtungseinheit in einem Liner verwendet werden.

Ein erfindungsgemäßes Verfahren zur Aushärtung eines erfindungsgemäßen lichthärtenden Liners oder eines erfindungsgemäßen lichthärtenden Kunststoffmaterials umfasst die folgenden Schritte:
- Bestrahlen des Liners (des Kunststoffmaterials) mit Licht eines ersten Wellenlängenbereiches, so dass eine Härtung des lichthärtenden Kunststoffmaterials (des Liners) bewirkt wird,
- Bestrahlen des Liners (des Kunststoffmaterials) mit Licht eines zweiten Wellenlängenbereiches zur Erfassung des Absorptions- und/oder Fluoreszenzverhaltens des im lichthärtenden Kunststoffmaterial enthaltenen Farbstoffs,
wobei der erste und der zweite Wellenlängenbereich vorzugsweise zumindest im Wesentlichen unterschiedlich sind und die Bestrahlung des Liners (des Kunststoffmaterials) mit Licht des ersten Wellenlängenbereichs in Abhängigkeit von dem durch Bestrahlen mit Licht des zweiten Wellenlängenbereichs erfassten Absorptions- und/oder Fluoreszenzverhalten des im lichthärtenden Kunststoffmaterial enthaltenen Farbstoffs gesteuert wird.

Üblicherweise kann das Spektrum der zur Lichtaushärtung genutzten Beleuchtungseinrichtungen (Lampen) unmittelbar zur Fluoreszenzanregung eines entsprechenden Farbstoffes genutzt werden. Die Nutzung zusätzlicher Lichtquellen ist deshalb üblicherweise nicht erforderlich. Da die Fluoreszenzemission beispielsweise des Rhodamin B (aber auch anderer Farbstoffe) in einem Spektralbereich stattfindet, in dem die üblicherweise eingesetzten Beleuchtungseinrichtung selbst nur mit geringen Intensität Licht emittieren, führt dies zu einem hohen Kontrast der Signale von nichtgehärtetem und gehärtetem Harz und somit zu einer sehr hohen Messempfindlichkeit des erfindungsgemäßen Verfahrens zur Bestimmung des Aushärtungsgrades eines lichthärtenden Kunstharzes.

Die Ergebnisse einer optischen Kontrolle der Lichtaushärtung stehen sowohl bei einer Farbmessung (Ausnutzung der Absorptionsänderung des Farbstoffes) wie auch bei der Methode der Fluoreszenzmessung in Echtzeit zur Verfügung; eine zusätzliche Materialprüfung ist daher nicht erforderlich.

Wenngleich die Erfindung insbesondere mit Blick auf Liner zur Auskleidung von (Alt-)Rohren erläutert wird, ist doch klar, dass das Prinzip der Indikation der Aushärtung eines lichthärtenden Kunstharzes mittels eines niedrigkonzentrierten Zusatzes eines Farbstoffes, dessen Absorptions- und/oder Fluoreszenzverhalten vom Aushärtungsgrad des Kunstharzes abhängt, auch in anderen technischen Gebieten einsetzbar, z.B. im Bereich der Zahnheilkunde, wo als Füllmaterial Kunststoffe eingesetzt werden, die durch Bestrahlung mit blauem Licht aushärten.

Die Erfindung wird nachfolgend anhand von Beispielen näher erläutert:

### Beispiel 1

Herstellung erfindungsgemäßer lichthärtender Kunststoffmaterialien:
1. Es wurden in separaten Ansätzen Lösungen folgender Farbstoffe in Aceton hergestellt:
   Rhodamin B
   Rhodamin 19
   Kresylviolett
   Nilblau
   Fluorescein.

   Die Konzentration der besagten Farbstoffe im Aceton betrug jeweils 0,8 ppt (Teile pro Tausend).
2. Jeweils etwa 150 µl der Lösung des jeweiligen Farbstoffes Aceton wurden in etwa 100 g eines in Styrol gelösten ungesättigten Polyesterharzes (UP-Harzes) Palatal P921-02 der Firma BASF eingemischt. Die Konzentration des Farbstoffs im UP-Harz betrug nach gründlicher Durchmischung etwa 1 ppm (Teile pro Million).

Anmerkung: Palatal P921-02 wird von verschiedenen Firmen für die Herstellung glasfaserverstärkter Kunststoffschläuche (GFK-Schlauch, sogenannter Reliner oder Inliner) verwendet.

Das erhaltene Gemisch aus Farbstoff und UP-Harz in Lösungsmitteln (Styrol/Aceton) war ein erfindungsgemäßes lichthärtbares Kunststoffmaterial, wobei die Aushärtung des Kunststoffmaterials durch einen Farbumschlag des jeweiligen Farbstoffs indiziert wird.

### Beispiel 2

Aushärtung erfindungsgemäßer lichthärtbarer Kunststoffmaterialien:

Die in Beispiel 1 hergestellten Kunststoffmaterialien wurden unter Verwendung einer Gasentladungslampe mit einer Füllung aus Gallium als Leuchtmittel und Quecksilber als Zündhilfe ausgehärtet. Die stärkste Gallium-Emissionslinie der Gasentladungslampe liegt bei 421 nm (blaues Licht) und liegt somit im gemäß Herstellerangaben wirksamsten Spektralbereich für die Aushärtung des UP-Harzes.

Die Gasentladungslampe wies überdies Emissionslinien bei 299 nm (Ga), 315-267-407-438-548-580 nm (Hg), 591 nm (Na) und 673 nm (Li) sowie ein Emissionskontinuum von 300 bis 450 nm auf, was für die Farbmessung bzw. die Fluoreszenzanregung der in den Kunststoffmaterialien gemäß Beispiel 1 enthaltenen Farbstoffe nützlich ist. Insbesondere können die Emissionslinien bei Wellenlängen oberhalb des für die Aushärtung wesentlichen Bereiches, insbesondere die besonders starken Linien der Gasentladungslampe bei 548, 580 und 591 nm zur Fluoreszenzanregung eines dort absorbierenden Farbstoffs genutzt werden.

Das jeweilige Kunststoffmaterial wurde so lange mittels der Gasentladungslampe bestrahlt, bis es ausgehärtet war.

Die Ergebnisse des Aushärtungsvorgangs sind in der nachfolgenden Tabelle 1 zusammengefasst:

**Tabelle 1**

| | Farbe Kunststoffmaterial vor Aushärtung | Farbe Kunststoffmaterial nach Aushärtung | Fluoreszenz vor Aushärtung | Fluoreszenz nach Aushärtung |
|---|---|---|---|---|
| Rhodamin B | rot | klar (farblos) | stark | gering |
| Rhodamin 19 | hellrot | klar (farblos) | stark | gering |
| Kresylviolett | dunkelrot | klar (farblos) | stark | gering |
| Nilblau | blau | klar (farblos) | stark | gering |
| Fluorescein | grüngelb | klar (farblos) | stark | gering |

Die Untersuchung zeigte, dass die genannten Farbstoffe hervorragend zur Indizierung der Aushärtung eines UP-Harzes geeignet sind. Rhodamin B zeigte den deutlichsten Farbumschlag (die deutlichste Verblassung).

### Beispiel 3

Indikation der Aushärtung eines lichthärtenden Liner-Materials:

Ein lichthärtendes Liner-Material wurde hergestellt, indem ein Glasfasergewebe (Glastyp: Aluminiumborosilikatglas, E-Glas für die Elektroindustrie), wie es üblicherweise zum Aufbau eine Laminats aus harzgetränktem Glasfasergewebe und beidseitig aufgebrachten transparenten Oberflächenfolien verwendet wird, mit analog Beispiel 2 hergestellten lichthärtenden Kunststoffmaterialien (UP-Harz mit eingemischtem Farbstoff gemäß Beispiel 1/2) imprägniert (durchtränkt) wurde.

Analog Beispiel 2 wurden die mit unterschiedlichen Farbstoffen versehenen harzgetränkten Glasfasermatten ausgehärtet.

Hinsichtlich Aushärtung und Farbstoffreaktion zeigten sich im Vergleich mit den reinen Kunststoffmaterialien gemäß Beispiel 2 zwar geänderte zeitliche Verläufe, jedoch keine wesentlichen Unterschiede hinsichtlich der erreichbaren Aushärtungsgrade und der Aushärtungsindikation.

Die erfindungsgemäßen lichthärtenden Kunststoffmaterialien sind somit zum Einsatz bei der Herstellung von Linern zur Auskleidung von Rohren geeignet. Die in den erfindungsgemäßen lichthärtenden Kunststoffmaterialien enthaltenen Farbstoffe sind hervorragend zur Indikation des Aushärtungsgrades eines jeweiligen Liners geeignet.

### Beispiel 4

Indikation der Aushärtung eines Komposits zur Verwendung als Kunststofffüllung in der Zahnheilkunde:

Analog zu üblichen Herstellungsverfahren wurde ein Komposit hergestellt, welches als Kunststoff-Füllung für eine Zahnfüllung eingesetzt werden könnte. Das Komposit enthielt einen lichthärtenden Kunststoff mit einem geringen Anteil an Rhodamin B.

Das Komposit besaß zunächst eine rote Farbe, die durch das Rhodamin B verursacht wurde.

Mittels einer im Bereich der Zahnheilkunde üblichen Beleuchtungsquelle wurde das Komposit ausgehärtet. Dabei verblasste die rote Farbe. Nach erfolgter Aushärtung entsprach die Farbe des Komposits der eines Vergleichskomposits ohne Rhodamin B-Anteil.

Erfindungsgemäße lichthärtende Kunststoffmaterialien sind folglich nicht nur zur Verwendung bei der Herstellung von Linern geeignet, sondern können in einer Vielzahl von Bereichen eingesetzt werden.

## Patentansprüche

1. Lichthärtendes Kunststoffmaterial, umfassend ein Gemisch aus:
- einem lichthärtenden Kunstharz und
- einem Farbstoff, dessen Absorptions- und/oder Fluoreszenzverhalten vom Aushärtungsgrad des Kunstharzes abhängen.

2. Lichthärtendes Kunststoffmaterial nach Anspruch 1,
**dadurch gekennzeichnet, dass** der Farbstoff im sichtbaren Bereich des elektromagnetischen Wellenspektrums absorbiert, wenn das lichthärtende Kunstharz im nicht ausgehärteten Zustand vorliegt.

3. Lichthärtendes Kunststoffmaterial nach einem der vorangehenden Ansprüche,
**dadurch gekennzeichnet, dass** der Farbstoff in einem Bereich des elektromagnetischen Wellenspektrums absorbiert, der zumindest im wesentlichen für die Lichthärtung des Kunstharzes nicht relevant ist.

4. Lichthärtendes Kunststoffmaterial nach einem der vorangehenden Ansprüche,
**dadurch gekennzeichnet, dass** der Farbstoff aus der Gruppe ausgewählt ist, die aus Rhodamin B, Rhodamin 19, Kresylviolett, Nilblau und Fluorescein besteht.

5. Lichthärtendes Kunststoffmaterial nach einem der vorangehenden Ansprüche,
**dadurch gekennzeichnet, dass** das lichhärtende Kunstharz ein Duroplast ist.

6. Lichthärtendes Kunststoffmaterial nach Anspruch 5,
**dadurch gekennzeichnet, dass** der Duroplast aus der Gruppe ausgewählt ist, die aus ungesättigtem Polyesterharz (UP-Harz) und Epoxidharz besteht

7. Lichthärtendes Kunststoffmaterial nach einem der vorangehenden Ansprüche,
**dadurch gekennzeichnet, dass** das Kunststoffmaterial einen Fotokatalysator für das lichthärtende Kunstharz umfasst.

8. Lichthärtendes Kunststoffmaterial nach Anspruch 7,
**dadurch gekennzeichnet, dass** der Fotokatalysator zur Katalyse der Aushärtungsreaktion des Kunstharzes in einem spektralen Bereich absorbiert, in dem der Farbstoff zumindest im wesentlichen nicht absorbiert.

9. Lichthärtender Liner zur Auskleidung von Rohren, umfassend
- ein Trägermaterial und
- ein lichthärtendes Kunststoffmaterial nach einem der vorangegangenen Ansprüche, umfassend ein Gemisch aus:
- einem lichthärtenden Kunstharz und
- einem Farbstoff, dessen Absorptions- und/oder Fluoreszenzverhalten vom Aushärtungsgrad des Kunstharzes abhängen,
wobei das Trägermaterial mit dem lichthärtenden Kunststoffmaterial imprägniert ist, so dass der Liner bei Aushärtung des Kunstharzes ebenfalls aushärtet.

10. System zur Auskleidung von Rohren, umfassend
- einen lichthärtenden kunstharzimprägnierten Liner nach Anspruch 9,
- eine oder mehrere Beleuchtungseinheiten zur Emission (a) von Strahlung eines ersten Wellenlängenbereiches zur Aushärtung des Liners und (b) von Strahlung eines zweiten Wellenlängenbereiches zur Absorption durch den Farbstoff des lichthärtenden Kunststoffmaterials und/oder zu dessen Fluoreszenz-Anregung,
- einen Empfänger für Informationen zum Absorptions- oder Fluoreszenzverhalten des lichthärtenden Kunststoffmaterials bei Bestrahlung mit Licht des zweiten Wellenlängenbereiches.

11. System nach Anspruch 10, weiter umfassend
- Steuerungsmittel, in denen die Informationen zum Absorptions- oder Fluoreszenzverhalten des lichthärtenden Kunststoffmaterials zur Steuerung der Aushärtung des Liners weiterverarbeitet werden.

12. Verwendung eines Farbstoffs als Indikator für den Aushärtungsgrad eines lichthärtenden Kunstharzes.

13. Verfahren zur Bestimmung des Aushärtungsgrades eines lichthärtenden Kunstharzes, wobei
- das Kunstharz im nicht ausgehärteten Zustand mit einem Farbstoff vermischt wird, dessen Absorptions- und/oder Fluoreszenzverhalten vom Aushärtungsgrad des Kunstharzes abhängen und
- das Absorptions- und/oder Fluoreszenzverhalten des Farbstoffs bestimmt werden.

14. Verfahren zur Aushärtung eines lichthärtenden Kunststoffmaterials nach einem der Ansprüche 1-8 oder eines lichthärtenden Liners nach Anspruch 9, umfassend die Schritte:
- Bestrahlen des Liners bzw. des lichthärtenden Kunststoffmaterials mit Licht eines ersten Wellenlängenbereichs, so dass eine Härtung des lichthärtenden Kunststoffmaterials bewirkt wird,
- Bestrahlen des Liners bzw. des lichthärtenden Kunststoffmaterials mit Licht eines zweiten Wellenlängenbereichs zur Erfassung des Absorptionsund/oder Fluoreszenzverhaltens des im lichthärtenden Kunststoffmaterial enthaltenen Farbstoffs,
wobei
der erste und der zweite Wellenlängenbereich zumindest im wesentlichen unterschiedlich sind und
die Bestrahlung des Liners bzw. des lichthärtenden Kunststoffmaterials mit Licht des ersten Wellenlängenbereichs in Abhängigkeit von dem durch Bestrahlen mit Licht des zweiten Wellenlängenbereichs erfassten Absorptions- und/oder Fluoreszenzverhalten des im lichthärtenden Kunststoffmaterial enthaltenen Farbstoffs gesteuert wird.

15. Verwendung eines lichthärtenden Kunststoffmaterials nach einem der Ansprüche 1 bis 8, zur Herstellung eines Komposits für die Füllung von Zahnkaritäten.
